# EUROPEAN PATENT SPECIFICATION

(11) **EP 2 863 411 B1**
(45) Date of publication and mention of the grant of the patent: **02.12.2020**
(21) Application number: 14188486.6
(22) Date of filing: 10.10.2014
(51) Int. Cl.: H01J 37/32

(54) **Plasma etching apparatus**
Plasmaätzvorrichtung
Appareil de gravure au plasma

(30) Priority: 15.10.2013 GB 201318249
(43) Date of publication of application: 22.04.2015
(73) Proprietor: SPTS Technologies Limited, Newport Gwent NP18 2TA (GB)
(72) Inventor: Varvara, Maxime, 74350 Copponex (FR)
(74) Representative: Wynne-Jones IP Limited

(56) References cited:
- EP-A1- 2 151 509
- EP-A2- 0 488 393
- JP-A- 2013 191 845
- US-A- 5 476 182
- US-A1- 2007 051 471

## Description

This invention relates to a plasma etching apparatus and to an associated kit of parts and a method of plasma etching a substrate.

Processes for the manufacture of devices on semiconductor wafers include a large number of plasma etch process steps which are carried out in a variety of plasma etch tools. Typically, plasma etch processes are used to selectively remove material from the areas of the wafer that are not covered by a mask. Etch depths can vary from nanometres to hundreds of microns in a variety of materials, such as silicon, GaAs, aluminium, and silicon dioxide. In all plasma etch processes there is a general requirement to provide a uniform, repeatable etch process. These qualities should be apparent both within a die (over an area of a few square millimetres or square centimetres) and across the entire wafer (currently to a diameter of 300 millimetres, although still large diameters may become the commercial norm in the future).

It is of course economically beneficial to be able to etch features as rapidly as possible due to the relatively high cost of plasma etch tools and clean rooms, and also because clean room floor space is at a premium, and therefore efficient utilisation of clean room space is important. Unfortunately, in practice in most cases a reduction in process times, through the use of high etch rates, results in a less uniform process performance. As such, it is typical for a compromise to be made so that both uniformity and etch rate are at acceptable levels. It is particularly important when etching deep features (tens to hundreds of microns) such as MEMS structures or through silicon vias (TSVs) in silicon, where long etch process times are required, for an optimal balance to be achieved between high etch rates and across wafer etch uniformity. To form these kinds of deep silicon etch features, the so-called 'Bosch process' of cyclic deposition/etch steps are typically used. The Bosch process is well-known in the art, and is described, for example, in US5501893.

Accordingly, a great deal of research has been carried out with the purpose of increasing etch rate without sacrificing etch uniformity. Representative examples in the prior art include US2006/0070703 and US7371332. The overwhelming received wisdom in the field is for plasma etch tools to use a process chamber where the internal diameter of the process chamber is considerably larger than the diameter of the wafer being processed. There is a technical explanation for this approach which also constitutes a received wisdom in the field. More specifically, it is considered beneficial for the process chamber to be of a considerably larger internal diameter than the diameter of the wafer being processed because it is believed that a uniform plasma is more readily achieved in such a system, with losses to the walls of the chamber - which result in non-uniformities in the plasma - occuring well away from the edge of the wafer being processed. Figure 1 of US2006/0070703 is a representative schematic diagram of a typical prior art single wafer ICP plasma etch system. As depicted in this figure, a cylindrical chamber has a central platen support or electrostatic chuck (ESC) that locates the circular wafer to be processed. A plasma is initiated and sustained by coupling RF power through an antenna, in this case a multi-turn coil, into the gas within the chamber. The gas enters at the top of the chamber and the by-products of the etch process exit the bottom of the chamber using a suitable pumping arrangement. The wafer platen can also be RF driven in certain system configurations in order to provide further control of the incident ions on the wafer surface as is well-known in the art. Although Figure 1 of US2006/0070703 is schematic, in fact the relative dimensions of the wafer to the chamber are essentially accurate representations of the prior art.

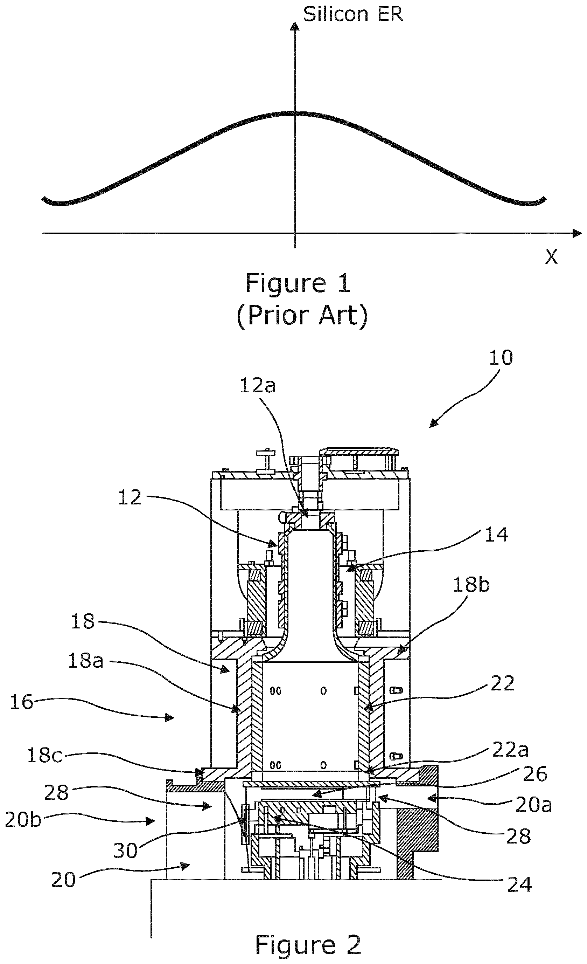

The present inventors have realised that when etching a wafer in a standard plasma etching chamber of the type shown in Figure 1 of US2006/0070703, a proportion of the active etchant gas completely bypasses the wafer by flowing down the sides of the chamber. The present inventors have also recognised that this is in efficient in terms of gas usage. Because pumping occurs below the wafer surface, the majority of the gas introduced into the chamber may never reach the wafer surface. For example, with a chamber of the type shown in Figure 1 of US2006/0070703 having a 350mm diameter with a 200mm diameter wafer in the centre of the chamber, around two thirds of the gas flow would exit directly to the pump. Additionally, the present inventors have realised that this configuration results in higher etch rate near to the centre of the wafer with lower etch rates being observed at the periphery of the wafer. This is represented schematically in Figure 1, which shows silicon etch rates as a function of position from the centre of the wafer. It can be seen that the prior art configuration promotes a centre high etch rate which essentially follows the gradient of etchant concentration as a function of distance from the centre of the chamber to the periphery of the wafer.

It is also known to provide two chamber plasma etching arrangements in which plasma generated in a first chamber flows into a second, processing, chamber in which the substrate resides. Again, it is received wisdom in the field for the internal diameter of the process chamber to be considerably larger than the diameter of the wafer being processed. Additionally, the present inventors have appreciated that the first chamber in which the plasma is generated is generally of a relatively large size in comparison to the diameter of the wafer. US2007/0158305 and EP2416351 both disclose what are effectively two chamber arrangements in which a guide, which may be a frusto-conical guide, is used to direct plasma towards a substrate located in the second chamber. However, the region of the first chamber in which the plasma is generated is of a diameter substantially greater than the diameter of the wafer being processed.

EP2151509A1 discloses a reactive gas distributor for a reactive gas treatment system, comprising a housing, a reactive gas inlet provided at one side of the housing and fluidly connectable to a remote plasma source, and a plurality of reactive gas outlets at another side of the housing and arranged in a pattern.

US5476182A discloses an apparatus for etching an insulating film of an object to be processed having the insulating film comprises a first chamber into which an inert gas is introduced, a plasma generating section for converting the inert gas to a plasma in the first chamber, a second chamber, which communicates with the first chamber, for receiving a reactive gas for etching the insulating film and generating radicals of the reactive gas therein, and a support electrode for supporting the object to be processed in the second chamber and attracting ions in the plasma of the inert gas to the object to be processed.

EP0488393A2 discloses a substrates treating apparatus comprising a chamber provided with a section at which a semiconductor wafer is treated and with another section at which plasma is generated, means for supplying mixed gas (O2 + CF4) into the plasma generating section in the chamber, high frequency electrodes for changing the gas into plasma, an ion trap for trapping ions in the plasma to send neutral radicals into the wafer treating section, and means (34) for exhausting the wafer treating section.

US2007/0051471A1 discloses an invention in which one embodiment is a stripping reactor that includes: (a) a remote plasma source disposed to output a gas; (b) a gas distribution plate connected to ground that transmits the gas output from the remote plasma source to a processing chamber; (c) a wafer support disposed in the processing chamber; (d) a wafer support assembly disposed about the wafer pedestal that includes an outer conductive peripheral structure connected to ground

JP2013191845A discloses a substrate treating apparatus includes an inner chamber in which a first space is formed, a first outer chamber surrounding the inner chamber and forming a second space separated from the first space, a susceptor disposed in the first space and adapted to support a substrate, a first exhaust line connected to the inner chamber and exhausting a gas in the first space to the outside, a process gas supply member adapted to supply a process gas for treating the substrate to the first space, and a monitor module disposed in the second space and adapted to monitor a state of the first space.

The present invention, in at least some of its embodiments, addresses one or more of the above described problems. In particular, at least some embodiments of the invention can provide improved etch uniformity and/or improved gas utilisation in comparison to a conventional system. Additionally, at least some embodiments of the invention can provide an improved etch rate in comparison to a conventional system.

For the avoidance of doubt, whenever reference is made herein to 'comprising' or 'including' and like terms, the invention is also understood to include more limiting terms such as 'consisting' and 'consisting essentially'.

According to a first aspect of the invention there is provided a plasma etching apparatus for plasma etching a substrate, the substrate including:
a first chamber having a plasma generation region, the plasma generation region having a cross-sectional area and shape;
a plasma generation device for generating a plasma in the plasma generation region;
a second chamber into which the plasma generated in the plasma generation chamber can flow, wherein the second chamber defines an interior having a cross-sectional area and shape, and the cross-sectional area of the interior is greater than the cross-sectional area of the plasma generation region;
a third chamber having a substrate support for supporting a substrate of the type having an upper surface to be plasma etched, wherein the third chamber has an interface with the second chamber so that the plasma, or one or more etchant species associated with the plasma, can flow from the second chamber to plasma etch the substrate;
   in which:
   the inner cross-sectional area and shape of the second chamber interior substantially corresponds to the upper surface of the substrate, the substrate and the interior of the second chamber each having at least one width, wherein the ratio of the width of the interior of the second chamber to the width of the substrate is in the range 1.15 to 1.0; and
   the substrate support is disposed so that, in use, the substrate is substantially in register with the interior of the second chamber, and the upper surface of the substrate is positioned at a distance of 80mm or less from the interface.

The ratio of the width of the interior of the second chamber to the width of the substrate may be in the range 1.1 to 1.0.

It will be appreciated that typically the substrate to be processed and the interior of the second chamber are of circular cross-section, in which instance the widths referred to above are diameters. In principle, the substrate to be processed and the interior of the second chamber may be of a different cross-sectional shape, and again in principle such a non-circular shape may have more than one characteristic width. In these embodiments, each characteristic width associated with the cross-sectional shape will have a corresponding ratio of the width of the interior of the second chamber to the width of the substrate. In these embodiments, each width ratio may satisfy the quantitative criteria above.

In some embodiments, the substrate support is disposed so that, in use, the upper surface of the substrate is positioned at a distance of 60mm or less from the interface.

In some embodiments, the substrate support is disposed so that, in use, the upper surface of the substrate is positioned at a distance of 10mm or more from the interface.

Preferably, the substrate support is disposed so that, in use, the upper surface of the substrate is positioned at a distance in the range 10-60mm from the interface.

The plasma generation region and the interior of the second chamber each have a cross-sectional area, The ratio of the cross-sectional area of the plasma generation region to the cross-sectional area of the second chamber may be in the range 0.07 to 0.7.

Typically, the first and second chambers are co-axial. In use, the substrate is typically also co-axial with the first and second chambers.

Typically, the first and second chambers are both of circular cross-section. Typically the substrate to be plasma etched is also of circular cross-section.

The first chamber may be of a bell jar shape, i.e., the first chamber may have a non-constant circular cross-section which varies as a function of position along a longitudinal axis of the chamber so as to flare out towards the second chamber.

The apparatus may further include a baffle disposed or near to the substrate support in order to channel a gas flow in the vicinity of substrate. The baffle may be disposed so as to, in use, increase a retention time of etchant species around a periphery of the wafer.

In some embodiments, the interface is defined by a spacer element disposed between the second chamber and the third chamber. The spacer element may be an annular element such as a ring. The use of a spacer element is convenient because it enables the distance between the upper surface of the substrate and the interface to be varied and fine-tuned.

Generally, the second chamber is not equipped with a plasma generation device. Instead, only the first chamber has an associated plasma generation device.

Typically, the substrate support is configured to support a substrate having a diameter of at least 200mm. Excellent results have been achieved with the present invention when applied to wafers having diameters of 200mm and 300mm.

The present invention is readily applicable to a great many etch materials and process gases, including but not limited to Si,GaAs, polymer, Al etch materials and fluorine, chlorine and oxygen based chemistries. The present invention may be applied to etching using the Bosch process of alternate etching and deposition steps.

The plasma generation device may be an ICP (inductively coupled plasma) source, a helion source, an ECR (electron cyclotron resonance) source, or any other convenient device.

Plasma etching apparatus of the invention may be provided as an original item of manufacture. Alternatively, an advantage of the present invention is that it is possible to retrofit existing plasma etching apparatus of the type wherein a processing chamber is provided having a cross-sectional area which is substantially greater than the area of the upper surface of the substrate to be etched.

The plasma etching apparatus may be provided in combination with the substrate, the substrate being supported by the substrate support. However, the invention pertains also the plasma etching apparatus when not in use or prior to use, i.e., without the substrate present on the substrate support.

According to a second aspect of the invention there is provided a kit for retrofitting an existing plasma etching apparatus in order to provide a retrofitted plasma etching apparatus according to the first aspect of the invention. The kit may include:
an adapter for connection to one or more portions of the existing plasma etching apparatus, the adapter including a sleeve which is configured to act as the second chamber when the adapter is connected, and further including connection means permitting the adapter to be connected to said one or more portions of the existing plasma etching apparatus to locate the sleeve in place.

The adapter may include one or more flange portions for connection to one or more portions of the existing plasma etching apparatus. The adapter may include an upper flange portion and a lower flange portion. The flange portions may form part of a structure which includes the sleeve. Alternatively the flange portions may form part of a structure in which the sleeve can be housed. This structure may include an outer sleeve for housing the sleeve which is configured to act as the second chamber.

The kit may further include a spacer element configured to be disposed underneath the sleeve and connectable to the adapter and/or the existing plasma etching apparatus so as to define the interface between the second chamber and the third chamber in the retrofitted plasma processing apparatus.

According to a third aspect of the invention there is provided a method of plasma etching a substrate including the steps of:
i) providing a plasma processing apparatus according to the first aspect of the invention;
ii) causing the substrate to be supported by the substrate support so that the substrate is substantially in register with the interior of the second chamber and the upper surface of the substrate is positioned at a distance of 80mm or less from the interface;
iii) generating a plasma in the plasma generation region; and
iv) causing the plasma, or one or more etchant species associated with the plasma, to etch the substrate.

Whilst the invention has been described above, it extends to any inventive combination of the features set out above, or in the following description, drawings or claims. For example, any features described in relation with one aspect of the invention is considered to be disclosed also in relation to any other aspect of the invention.

Embodiments of apparatus and methods in accordance with the invention will now be described with reference to the accompanying drawings, in which:-
Figure 1 shows silicon etch rate as a function of distance from a silicon wafer centre during etching using a conventional apparatus;
Figure 2 is a cross-sectional view of an etching apparatus which has been retrofitted to provide a plasma etching apparatus of the invention;
Figure 3 is a perspective view of the interface region between the second and third chambers of the plasma etching apparatus of Figure 2;
Figure 4 shows etch depth as a function of position on a 300mm silicon wafer;
Figure 5 shows silicon etch rate as a function of position on a 200mm silicon wafer;
Figure 6 shows silicon etch rate and etch depth uniformity as a function of the diameter of the second chamber when etching 200mm silicon wafers; and
Figure 7 shows silicon etch rate and etch depth uniformity as a function of the gap between the second chamber and the wafer upper surface when etching 200mm diameter silicon wafers.

Figure 2 depicts plasma etching apparatus, shown generally at 10, of the invention. The embodiment shown in Figure 2 is in fact a commercially available plasma etching apparatus which has been retrofitted to produce apparatus in accordance with the present invention. More specifically, the apparatus shown in Figure 2 is a retrofit of plasma etching apparatus produced by the applicants and marketed under the trade name DSi. The apparatus 10 comprises a first chamber 12 in the form of a ceramic bell jar having a gas inlet 12a through which gases are introduced in order to produce plasma. A portion of the first chamber 12 is surrounded by an ICP source 14 which is used to initiate and sustain a plasma in at least a plasma generation region of the first chamber 12 in a manner which is well-known to those skilled in the art. The lower end of the first chamber 12 flares out into an intermediate portion of the apparatus 10. The intermediate portion is depicted generally at 16 in Figure 2, and is the main retrofitted component in the apparatus 10. The intermediate portion 16 includes an adapter structure 18 having a sleeve 18a, upper flange portion 18b and lower flange portion 18c. The upper flange portion is connected to the first chamber 12 and other upper portions of the apparatus 10. The lower flange portion is connected to a third chamber 20. The sleeve 18a is sized to carry a reduced-diameter second chamber 22 in the form of a sleeve which is positioned and located within the sleeve 18a. The second chamber 22 can further comprise a lower ring 22a which in this embodiment is connected to the sleeve 22. Below the intermediate section including the second chamber there is the third chamber 20 which houses an electrostatic chuck (ESC) 24 for supporting a wafer 26 to be processed. The third chamber 20 includes a slot valve 20a for introducing the wafer 26 to the apparatus 10 and for removing same. The third chamber further includes an outlet 20b. Gases exit the apparatus from the outlet 20b using a suitable pumping arrangement (not shown) as is well-known to the skilled reader. It is noted that Figure 2 does not show a complete view of the third chamber. Instead, Figure 2 only shows an upper portion of the third chamber. The internal diameter of the third chamber 20 is of necessity considerably larger than the diameter of the wafer in order to enable the wafer to be introduced and removed from the apparatus 10. A cylindrical cover 28 is disposed around an upper portion of the apparatus 10 including the first and second chambers 12, 22 for safety purposes.

A baffle 28 is provided around the ESC 24 and a wafer 26 in order to increase the retention time of the etchant gas around the periphery of the wafer 26. A wafer edge protection (WEP) arrangement 30 is also provided.

In the conventional DSi apparatus, a different cylindrical structure serves as the second chamber, and its internal diameter is significantly greater than the diameter of the wafer. In the present invention, the internal diameter of the sleeve 22 (and the ring 22a) are matched to the diameter of the wafer to be processed. In a representative example, the wafer is a 200mm diameter and the internal diameter of the sleeve 22 and ring 22a is also 200mm. As described elsewhere herein, it is not mandatory that these diameters should correspond exactly, although advantageous results have been achieved with such an exact matching of the diameters. It will be appreciated that when the wafer 26 is mounted on the ESC 24, the wafer 26 is in register with the second chamber 22.

Examples of improved etching are now described using the apparatus shown in Figure 2. Etching was performed in accordance with the Bosch process.

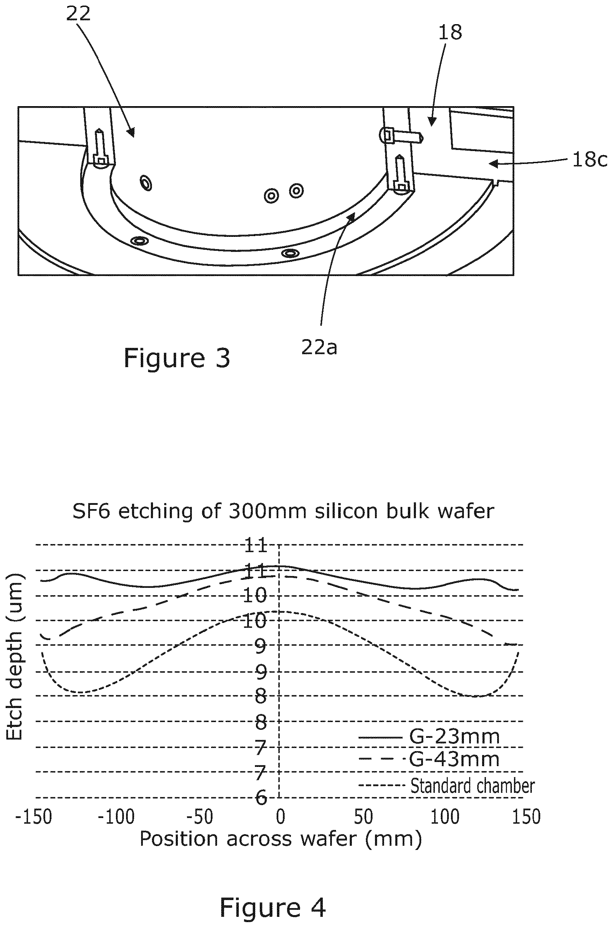

In Figure 4 we show the improvements in process performance achieved in etch rate and uniformity for a Si etch process on 300mm diameter wafers using a SF₆ chemistry. By reducing the size (ID) of the second chamber from the standard 350mm to 300mm while maintaining a chamber to wafer gap of 43 and 23mm, etch rates increase to 9.8 and 10.3mm/min, respectively, while uniformity is also significantly improved over the standard value of 9.7%. The results are summarized in Table 1.

**Table 1. Silicon ER (microns/min) and uniformity values for 300mm silicon bulk wafer etched with a SF₆ plasma; standard (350mm ID) and reduced diameter (G=300mm ID).**

| **Gap** | **Etch rate [µm/min]** | **Uniformity [±%]** |
|---|---|---|
| G-23mm | 10.3 | 2.6 |
| G-43mm | 9.8 | 7.4 |
| Standard chamber | 8.8 | 9.7 |

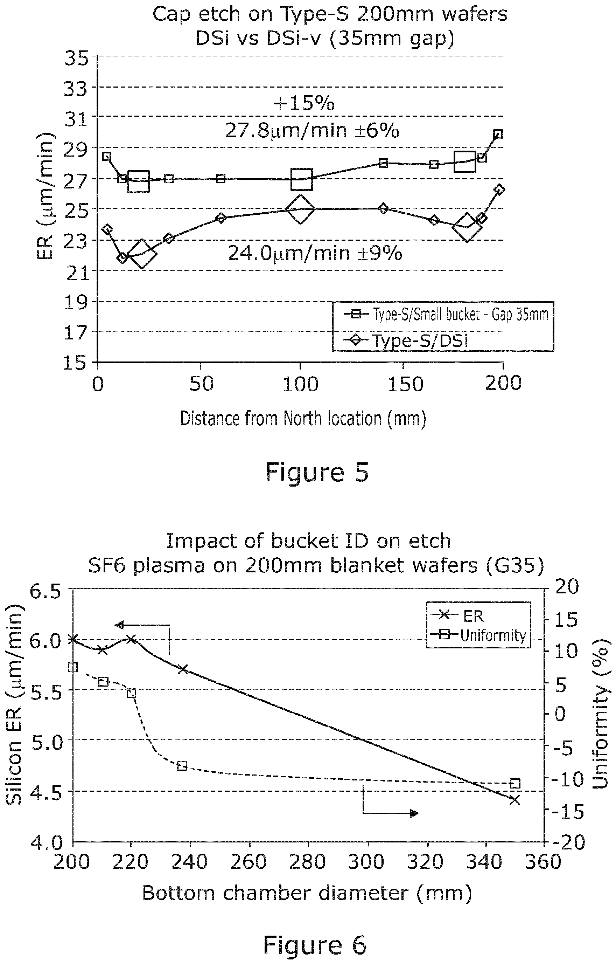

In Figures 5, 6 and 7 we can see representative results for 200mm diameter wafers with a second chamber ID of ∼200mm. A substantial improvement is seen in all cases when the 200mm second ID chamber (with a 35mm gap between the second chamber and the wafer) is compared with the standard 350mm ID second chamber.

In Figure 5 we can see a 15% improvement in etch rate for a Bosch Si etch process on patterned Si wafers between the standard chamber and the reduced diameter second chamber. Uniformity is also improved from +/- 9% with the standard chamber to +/- 6% with the smaller second chamber of the invention.

In Figure 6 we can see the Si etch rate and uniformity for 200mm diameter Si wafers as a function of the second chamber internal diameter with a fixed gap between the second chamber and the wafer of 35mm. At ∼220-235 mm there is a large reduction in uniformity coupled with a more gradual decrease in etch rate as one moves towards larger second chamber IDS.

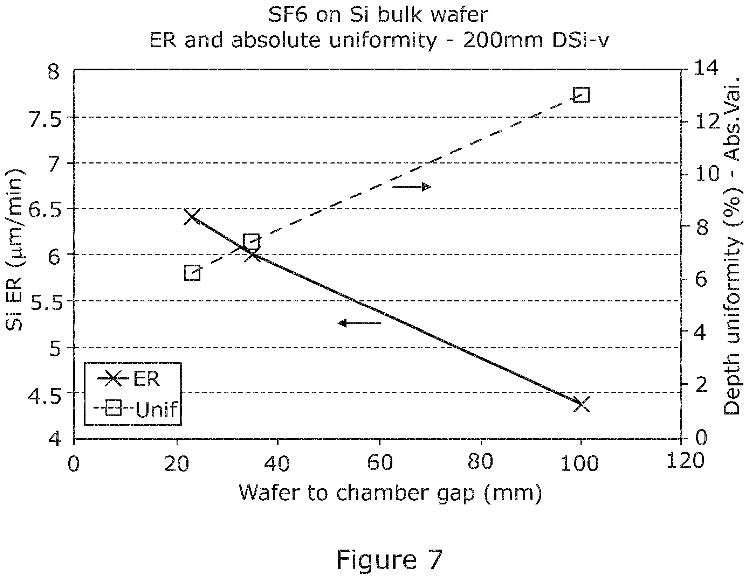

The importance of close coupling of the small lower chamber with the wafer is established in Figure 7 where a Si etch process is conducted on 200mm diameter wafers over a range (23-100mm) of second chamber to wafer gaps. The optimum values for etch rate and uniformity are with the smallest gaps.

Without wishing to be bound by any particular theory or conjecture, it is believed that the advantageous properties described herein can be attributed to the combination of three factors. Firstly, the cross-sectional area of the interior of the second chamber is greater than the cross-sectional area of the first chamber, at least in the region where the plasma is generated. In this way, the volume in which the plasma is initially generated is not too large, and a relatively uniform initial plasma can be formed. In contrast, relatively large plasma generation chambers can give rise toroidally distributed plasmas. It is believed that if the initially generated plasma is not very uniform, then it is at best difficult to provide subsequent processing steps which result in uniform etching. Secondly, the diameter of the second chamber should be close to the diameter of the wafer. This is surprising, since it goes against the received wisdom in the art. In the unlikely but theoretical event that the wafer is not of circular cross-section, then the second chamber should be of a similar shape which closely matches the characteristic dimensions of the wafer. Thirdly, the gap between the wafer (in its in-use position during etching) and the closely matching second chamber should be small.

The apparatus provided by the invention can improve the gas and plasma containment above the plane of the wafer compared to prior art chambers of larger ID. The present invention can avoid or at least reduce the loss of etchant gas going directly to the pumping line, increase the etching rate, and/or improve the cross-wafer depth uniformity. Again, without wishing to be bound by any particular theory or conjecture, it is believed that the present invention can force the etchant gas to interact with the wafer around the wafer periphery before being pumped away. In practice, a balance should be found between this mixing and the reduced conductance that can be caused for pumping the etch products away from the wafer. A baffle might be provided around or in close proximity to the wafer to assist in this regard. However, the use of a baffle is not an essential feature of the invention. The skilled reader will realise that the invention can be implemented and optimised in many different ways, and such variations are within the scope of the invention. For example, it is not necessary that the wafer is supported by an ESC, or that a WEP arrangement is used. Also, instead of retrofitting an existing apparatus, it is possible to produce a new plasma etching apparatus in accordance with the invention. The third chamber may be pumped from a port located at the bottom of the chamber, instead of the side of the chamber. Other plasma generation devices might be contemplated.

## Claims

1. A plasma etching apparatus (10) for plasma etching a substrate, the apparatus including:
a first chamber (12) having a plasma generation region, the plasma generation region having a cross-sectional area and shape;
a plasma generation device (14) for generating a plasma in the plasma generation region;
a second chamber (22) into which the plasma generated in the plasma generation chamber can flow, wherein the second chamber defines an interior having a cross-sectional area and shape, and the cross-sectional area of the interior is greater than the cross-sectional area of the plasma generation region;
a third chamber (20) having a substrate support (24) for supporting a substrate of the type having an upper surface to be plasma etched, wherein the third chamber has an interface with the second chamber so that the plasma, or one or more etchant species associated with the plasma, can flow from the second chamber to etch the substrate;
in which:
the inner cross-sectional area and shape of the second chamber interior substantially corresponds to the upper surface of the substrate, the substrate and the interior of the second chamber each having at least one width, **characterised in that** the ratio of the width of the interior of the second chamber to the width of the substrate is in the range 1.15 to 1.0; and
the substrate support is disposed so that, in use, the substrate is substantially in register with the interior of the second chamber, and the upper surface of the substrate is positioned at a distance of 80mm or less from the interface.

2. A plasma etching apparatus according to claim 1 in which the ratio of the width of the interior of the second chamber to the width of the substrate is in the range 1.1 to 1.0.

3. A plasma etching apparatus according to any previous claim in which the substrate support is disposed so that, in use, the upper surface of the substrate is positioned at a distance of 60mm or less from the interface.

4. A plasma etching apparatus according to any previous claim in which the substrate support is disposed so that, in use, the upper surface of the substrate is positioned at a distance of 10mm or more from the interface.

5. A plasma etching apparatus according to any previous claim in which the ratio of the cross-sectional area of the plasma generation region to the cross-sectional area of the second chamber is in the range 0.07 to 0.7.

6. A plasma etching apparatus according to any previous claim in which the first and second chambers are co-axial.

7. A plasma etching apparatus according to any previous claim in which the first and second chambers are both of circular cross-section.

8. A plasma etching apparatus according to any previous claim in which the first chamber is of a bell jar shape.

9. A plasma etching apparatus according to any previous claim in which the interface is defined by a spacer element (22a) disposed between the second chamber and the third chamber.

10. A plasma etching apparatus according to any previous claim further including a baffle (28) disposed on or near to the substrate support in order to channel a gas flow in the vicinity of the substrate.

11. A plasma etching apparatus according to any previous claim in combination with the substrate (26), the substrate being supported by the substrate support.

12. A kit for retrofitting an existing plasma etching apparatus in order to provide a retrofitted plasma etching apparatus according to claim 1, the kit including:
an adapter (18) for connection to one or more portions of the existing plasma etching apparatus, the adapter including a sleeve (22) which is configured to act as the second chamber when the adapter is connected, and further including connection means (18b, 18c) permitting the adapter to be connected to said one or more portions of the existing plasma etching apparatus to locate the sleeve in place.

13. A kit according to claim 12 in which the adapter includes one or more flange portions (18b, 18c) for connection to one or more portions of the existing plasma etching apparatus.

14. A method of plasma etching a substrate including the steps of:
i. providing a plasma etching apparatus according to claim 1;
ii. causing the substrate to be supported by the substrate support so that the substrate is substantially in register with the interior of the second chamber and the upper surface of the substrate is positioned at a distance of 80mm or less from the interface;
iii. generating a plasma in the plasma generation region; and
iv. causing the plasma, or one or more etchant species associated with the plasma, to etch the substrate.

## Patentansprüche

1. Plasmaätzvorrichtung (10) zum Plasmaätzen eines Substrats, wobei die Vorrichtung Folgendes umfasst:
eine erste Kammer (12) mit einem Plasmaerzeugungsbereich, wobei der Plasmaerzeugungsbereich eine Querschnittsfläche und -form aufweist;
eine Plasmaerzeugungsvorrichtung (14) zum Erzeugen eines Plasmas in dem Plasmaerzeugungsbereich;
eine zweite Kammer (22), in die das in der Plasmaerzeugungskammer erzeugte Plasma einströmen kann, wobei die zweite Kammer einen Innenraum mit einer Querschnittsfläche und -form definiert und die Querschnittsfläche des Innenraums größer ist als die Querschnittsfläche des Plasmaerzeugungsbereichs;
eine dritte Kammer (20) mit einem Substratträger (24) zum Tragen eines Substrats des Typs mit einer zu plasmaätzenden oberen Oberfläche, wobei die dritte Kammer eine Grenzfläche mit der zweiten Kammer aufweist, so dass das Plasma oder eine oder mehrere dem Plasma zugeordnete Ätzmittelspezies aus der zweiten Kammer fließen können, um das Substrat zu ätzen;
wobei:
die innere Querschnittsfläche und -form des Innenraums der zweiten Kammer im Wesentlichen der oberen Oberfläche des Substrats entspricht, wobei das Substrat und das Innere der zweiten Kammer jeweils mindestens eine Breite aufweisen, **dadurch gekennzeichnet, dass**
das Verhältnis der Breite des Innenraums der zweiten Kammer zur Breite des Substrats im Bereich von 1,15 bis 1,0 liegt; und
der Substratträger so angeordnet ist, dass das Substrat im Gebrauch im Wesentlichen mit dem Inneren der zweiten Kammer ausgerichtet ist, und die Oberseite des Substrats sich in einem Abstand von höchstens 80 mm von der Grenzfläche befindet.

2. Plasmaätzvorrichtung nach Anspruch 1, bei der das Verhältnis der Breite des Innenraums der zweiten Kammer zur Breite des Substrats im Bereich von 1,1 bis 1,0 liegt.

3. Plasmaätzvorrichtung nach einem der vorhergehenden Ansprüche, bei der der Substratträger so angeordnet ist, dass im Gebrauch die obere Oberfläche des Substrats in einem Abstand von 60 mm oder weniger von der Grenzfläche positioniert ist.

4. Plasmaätzvorrichtung nach einem der vorhergehenden Ansprüche, bei der der Substratträger so angeordnet ist, dass im Gebrauch die obere Oberfläche des Substrats in einem Abstand von 10 mm oder mehr von der Grenzfläche positioniert ist.

5. Plasmaätzvorrichtung nach einem der vorhergehenden Ansprüche, bei der das Verhältnis der Querschnittsfläche des Plasmaerzeugungsbereichs zur Querschnittsfläche der zweiten Kammer im Bereich von 0,07 bis 0,7 liegt.

6. Plasmaätzvorrichtung nach einem der vorhergehenden Ansprüche, bei der die erste und die zweite Kammer koaxial angeordnet sind.

7. Plasmaätzvorrichtung nach einem der vorhergehenden Ansprüche, bei der die erste und die zweite Kammer beide einen kreisförmigen Querschnitt aufweisen.

8. Plasmaätzvorrichtung nach einem der vorhergehenden Ansprüche, bei der die erste Kammer eine Glockenform aufweist.

9. Plasmaätzvorrichtung nach einem der vorhergehenden Ansprüche, bei der die Grenzfläche durch ein Abstandselement (22a) definiert ist, das zwischen der zweiten Kammer und der dritten Kammer angeordnet ist.

10. Plasmaätzvorrichtung nach einem der vorhergehenden Ansprüche, die ferner eine Prallfläche (28) umfasst, die auf oder in der Nähe des Substratträgers angeordnet ist, um einen Gasstrom in der Nähe des Substrats zu leiten.

11. Plasmaätzvorrichtung nach einem der vorhergehenden Ansprüche in Kombination mit dem Substrat (26), wobei das Substrat von dem Substratträger getragen wird.

12. Kit zum Nachrüsten einer vorhandenen Plasmaätzvorrichtung, um eine nachgerüstete Plasmaätzvorrichtung nach Anspruch 1 bereitzustellen, wobei der Kit Folgendes umfasst:
einen Adapter (18) zum Verbinden mit einem oder mehreren Abschnitten der vorhandenen Plasmaätzvorrichtung, wobei der Adapter eine Hülse (22) umfasst, die dafür konfiguriert ist, als die zweite Kammer zu wirken, wenn der Adapter verbunden ist, und wobei der Kit ferner Verbindungsmittel (18b, 18c) umfasst, die es dem Adapter ermöglichen, mit dem einen oder den mehreren Abschnitten der vorhandenen Plasmaätzvorrichtung verbunden zu werden, um die Hülse an Ort und Stelle zu lokalisieren.

13. Kit nach Anspruch 12, bei dem der Adapter einen oder mehrere Flanschteile (18b, 18c) zur Verbindung mit einem oder mehreren Teilen der vorhandenen Plasmaätzvorrichtung umfasst.

14. Verfahren zum Plasmaätzen eines Substrats, das folgende Schritte umfasst:
i. Bereitstellen einer Plasmaätzvorrichtung nach Anspruch 1;
ii. Bewirken, dass das Substrat von dem Substratträger getragen wird, so dass das Substrat im Wesentlichen in Ausrichtung mit dem Inneren der zweiten Kammer angeordnet ist und die obere Oberfläche des Substrats in einem Abstand von 80 mm oder weniger von der Grenzfläche positioniert ist;
iii. Erzeugen eines Plasmas in dem Plasmaerzeugungsbereich; und
iv. Bewirken, dass das Plasma oder eine oder mehrere mit dem Plasma assoziierte Ätzspezies das Substrat ätzen.

## Revendications

1. Appareil de gravure au plasma (10) servant à des fins de gravure au plasma d'un substrat, l'appareil comprenant :
une première chambre (12) ayant une région de génération de plasma, la région de génération de plasma ayant une surface et une forme de section transversale ;
un dispositif de génération de plasma (14) servant à générer un plasma dans la région de génération de plasma ;
une deuxième chambre (22) dans laquelle le plasma généré dans la chambre de génération de plasma peut s'écouler, dans lequel la deuxième chambre définit une partie intérieure ayant une surface et une forme de section transversale, et la surface de section transversale de la partie intérieure est supérieure à la surface de section transversale de la région de génération de plasma ;
une troisième chambre (20) ayant un support de substrat (24) servant à supporter un substrat du type ayant une surface supérieure destinée à être gravée au plasma, dans lequel la troisième chambre a une interface avec la deuxième chambre de telle sorte que le plasma, ou une ou plusieurs espèces d'agents de gravure associées au plasma, peut s'écouler en provenance de la deuxième chambre pour graver le substrat ;
dans lequel :
la surface et la forme de section transversale intérieure de la partie intérieure de la deuxième chambre correspondent sensiblement à la surface supérieure du substrat, le substrat et la partie intérieure de la deuxième chambre ayant chacun au moins une largeur, **caractérisé en ce que**
le rapport entre la largeur de la partie intérieure de la deuxième chambre et la largeur du substrat se trouve dans la plage allant de 1,15 à 1,0 ; et
le support de substrat est disposé de telle sorte que, lors de l'utilisation, le substrat est sensiblement calé sur la partie intérieure de la deuxième chambre, et la surface supérieure du substrat est positionnée à une distance de 80 mm ou moins par rapport à l'interface.

2. Appareil de gravure au plasma selon la revendication 1, dans lequel le rapport entre la largeur de la partie intérieure de la deuxième chambre et la largeur du substrat se trouve dans la plage allant de 1,1 à 1,0.

3. Appareil de gravure au plasma selon l'une quelconque des revendications précédentes, dans lequel le support de substrat est disposé de telle sorte que, lors de l'utilisation, la surface supérieure du substrat est positionnée à une distance de 60 mm ou moins par rapport à l'interface.

4. Appareil de gravure au plasma selon l'une quelconque des revendications précédentes, dans lequel le support de substrat est disposé de telle sorte que, lors de l'utilisation, la surface supérieure du substrat est positionnée à une distance de 10 mm ou plus par rapport à l'interface.

5. Appareil de gravure au plasma selon l'une quelconque des revendications précédentes, dans lequel le rapport entre la surface de section transversale de la région de génération de plasma et la surface de section transversale de la deuxième chambre se trouve dans la plage allant de 0,07 à 0,7.

6. Appareil de gravure au plasma selon l'une quelconque des revendications précédentes, dans lequel les première et deuxième chambres sont co-axiales.

7. Appareil de gravure au plasma selon l'une quelconque des revendications précédentes, dans lequel les première et deuxième chambres sont toutes les deux d'une coupe transversale circulaire.

8. Appareil de gravure au plasma selon l'une quelconque des revendications précédentes, dans lequel la première chambre est en forme de cloche.

9. Appareil de gravure au plasma selon l'une quelconque des revendications précédentes, dans lequel l'interface est définie par un élément d'espacement (22a) disposé entre la deuxième chambre et la troisième chambre.

10. Appareil de gravure au plasma selon l'une quelconque des revendications précédentes, comprenant par ailleurs un déflecteur (28) disposé sur le support de substrat ou près de celui-ci afin de canaliser un flux de gaz à proximité du substrat.

11. Appareil de gravure au plasma selon l'une quelconque des revendications précédentes en combinaison avec le substrat (26), le substrat étant supporté par le support de substrat.

12. Kit de modification en rattrapage d'un appareil de gravure au plasma existant à des fins de mise en œuvre d'un appareil de gravure au plasma modifié en rattrapage selon la revendication 1, le kit comprenant :
un adaptateur (18) servant à des fins de raccordement à une ou plusieurs parties de l'appareil de gravure au plasma existant, l'adaptateur comprenant un manchon (22) qui est configuré pour tenir lieu de deuxième chambre quand l'adaptateur est raccordé, et comprenant par ailleurs un moyen de raccordement (18b, 18c) permettant à l'adaptateur d'être raccordé auxdites une ou plusieurs parties de l'appareil de gravure au plasma existant pour positionner le manchon en place.

13. Kit selon la revendication 12, dans lequel l'adaptateur comprend une ou plusieurs parties formant bride (18b, 18c) servant à des fins de raccordement à une ou plusieurs parties de l'appareil de gravure au plasma existant.

14. Procédé de gravure au plasma servant à graver un substrat comprenant les étapes consistant à :
i. mettre en œuvre un appareil de gravure au plasma selon la revendication 1 ;
ii. amener le substrat à être supporté par le support de substrat de telle sorte que le substrat est sensiblement calé sur la partie intérieure de la deuxième chambre et la surface supérieure du substrat est positionnée à une distance de 80 mm ou moins par rapport à l'interface ;
iii. générer un plasma dans la région de génération de plasma ; et
iv. amener plasma, ou une ou plusieurs espèces d'agents de gravure associées au plasma, à graver le substrat.
